# EUROPÄISCHE PATENTSCHRIFT

(11) **EP 0 867 167 B1**
(45) Veröffentlichungstag und Bekanntmachung des Hinweises auf die Patenterteilung: **02.05.2001**
(21) Anmeldenummer: 98100091.2
(22) Anmeldetag: 07.01.1998
(51) Int. Cl.: A61K 7/00, A61K 7/06

(54) **Haarbehandlungsmittel und Verfahren zur Haarbehandlung**
Hair treatment composition and process for its use
Agent de soin capillaire et procédé d'utilisation

(30) Priorität: 04.02.1997 DE 19704038
(43) Veröffentlichungstag der Anmeldung: 30.09.1998
(73) Patentinhaber: GOLDWELL GmbH, 64297 Darmstadt (DE)
(72) Erfinder: Cajan, Christine, 56130 Bad Ems (DE); Gregov, Nico, 64291 Darmstadt (DE)

(56) Entgegenhaltungen:
- EP-A- 0 468 703
- GB-A- 2 180 215
- US-A- 4 886 660
- US-A- 5 059 414

## Beschreibung

Die vorliegende Erfindung betrifft ein Haarbehandlungsmittel, insbesondere zum Konditionieren von Haaren, das verbesserte anwendungstechnische Eigenschaften aufweist und den Haaren vor allem Glanz und Festigkeit verleiht.

Haarfestiger- und Styling-Produkte sind seit langem bekannt und in vielfachen Ausgestaltungen auf dem Markt erhältlich.

Sie können als Lösungen, Emulsionen, Gele, Haarsprays und Aerosolschäume eingesetzt werden.

Darüber hinaus existieren auch hochviskose Haarpflegeprodukte, die in einer Öl/Wasser-Grundlage vorliegen, und zumeist weitere haarpflegende Stoffe wie beispielsweise langkettige quaternäre Ammoniumverbindungen enthalten.
Diese Produkte werden als Nachbehandlungsmittel in das Haar einmassiert und verleihen diesem insbesondere erhöhten Glanz; sie werden gemeinhin als Gelwachs bezeichnet.

Aus der EP-A 468 703 ist ein Shampoo-System bekannt, das aus einer Doppelpackung besteht, deren einer Teil eine Zusammensetzung aus Tensiden und kationischen Polymeren, und deren anderer Teil ein Shampoo-Additiv mit einer Teilchengröße von ≤ 2 um in wäßriger Grundlage enthält. Der Viskositätsbereich von Shampoos liegt üblicherweise zwischen etwa 2.500 und 12.000 mPa•s bei 25 °C.

Die US 50 59 414 A beschreibt die Anwendung von zwei bis zur Anwendung voneinander getrennt gehaltenen Produkten in einem Behälter zur gemeinsamen Abgabe und Vermischen bei der Anwendung, wobei durch die Vermischung beider Phasen ein Endprodukt mit hoher Viskosität erhalten werden soll; ein der erfindungsgemäßen Wirkung entgegengesetzter Effekt.

Es wurde nun gefunden, daß man ein Haarbehandlungsmittel erhält, das den an beide obengenannten Produkte gestellten Anforderungen gleichzeitig gerecht wird, d.h., dem Haar konditionierende bzw. festigende Eigenschaften einerseits und Glanz andererseits verleiht und darüberhinaus leicht auf das Haar auftragbar ist, wenn dieses Haarbehandlungsmittel aus zwei bis zur Anwendung voneinander getrennt gehaltenen Zusammensetzungen besteht, von denen die Zusammensetzung A in wäßriger oder wäßrig-alkoholischer Grundlage mindestens ein filmbildendes synthetisches und/oder natürliches Polymer enthält und eine Viskosität zwischen etwa 25 000 und etwa 150 000, insbesondere 50 000 und 125 000 mPa.s bei 20°C (gemessen im Brookfield-Viskosimeter mit einer Spindel T-D bei 5 UpM) aufweist, und die davon bis zur Anwendung getrennt gehaltene Zusammensetzung B in Form einer gelförmigen. Fette bzw. Öle und Emulgator(en) enthaltenden wäßrigen Emulsion, die eine Viskosität zwischen etwa 300 000 und 1 000 000, insbesondere 400 000 und 800 000, besonders bevorzugt 500 000 und 700 000 mPa.s bei 20°C (gemessen im Brookfield-Viskosimeter mit einer Spindel T-E bei 5 UpM), aufweist, vorliegt, wobei beim Vermischen etwa gleicher Anteile beider Zusammensetzungen ein niederviskoses Produkt mit einer Viskosität zwischen etwa 100 und etwa 1500, vorzugsweise 250 bis 1000, insbesondere 300 bis 700 mPa.s bei 20°C (gemessen im Brookfield-Viskosimeter mit einer Spindel CP-51 bei 20UpM) erhalten

Dadurch ist es möglich, das erhaltene Endprodukt gut auf der Hand zu verteilen und entsprechend auf das Haar aufzubringen und einzumassieren, wobei gleichzeitig Festigung und ein diskreter Glanz erzielt werden.

Gegenstand der Erfindung ist daher auch ein Verfahren zur Haarbehandlung, insbesondere zur Haarkonditionierung und -festigung sowie zur Verleihung von Haarglanz, das dadurch gekennzeichnet ist, daß eine wie oben definierten mittelviskose Zusammensetzung A mit einer wie oben definierten hochviskosen Zusammensetzung B vermischt und die so erhaltene niedrigviskose Mischung auf das Haar aufgebracht und dort gleichmäßig verteilt wird.

Durch das erfindungsgemäße Mittel und das unter Benutzung desselben angewandte Verfahren wird eine Haarkonditionierung erreicht, die bisher nur durch aufeinanderfolgende Applikation von mindestens zwei unterschiedlichen Produkten erreicht wurde.

Darüber hinaus ermöglicht die erfindungsgemäße Behandlung auch eine gründlichere und gleichmäßigere Verteilung des durch Vermischen erhaltenen, niederviskosen Kombinationsproduktes auf dem Haar als es bisher mit den höherviskosen Einzelprodukten möglich war.

Zusammensetzung A, ein Haarfestiger, enthält in wäßriger oder wäßrig-alkoholischer Grundlage als essentiellen Bestandteil mindestens ein filmbildendes natürliches oder synthetisches Polymer.

Bevorzugte Polymere sind hierbei die bekannten nichtionischen Polymeren, insbesondere Vinylpyrrolidon/Vinylacetat- und/oder Vinylpropionat-Copolymere, wie sie insbesondere unter dem Handelsnamen "Luviskol^{R}" auf dem Markt erhältlich sind.

Weitere nichtionische Polymere sind Polyvinylpyrrolidon selbst, (Meth-)Acrysäureester-Copolymere, Polyacrylamid mit höherem Molekulargewicht, Chitosan und abgewandelte Chitosan-Derivate, Dimethylhydantoin-Formaldehyd-Harze, etc.

Es können natürlich auch Gemische aus verschiedenen nichtionischen Polymeren eingesetzt werden, ebenso Gemische aus diesen und kationischen, amphoteren und/oder anionischen Polymeren.

Kationische Polymere, die allein oder im Gemisch mit anderen Polymeren eingesetzt werden können, sind neben den altbekannten quaternären Cellulosederivaten des Typs "Polymer^{R}JR" insbesondere quaternisierte Homo- und Copolymere des Dimethyldiallylamoniumchlorids, wie sie unter dem Namen "Merquat^{R}" im Handel sind, quaternäre Vinylpyrrolidon-Copolymere, insbesondere mit Dialkylaminoalkyl(meth)acrylaten, unter dem Namen "Gafquat^{R}" bekannt, Copolymerisate aus Vinylpyrrolidon und Vinylimidazoliniummethochlorid, die unter dem Handelsnamen "Luviquat^{R}" angeboten werden, Polyamino-Polyamid-Derivate, beispielsweise Copolymere von Adipinsäure-Dimethylaminohydroxypropyldiethylentriamin, wie sie unter dem Namen "Cartaretine^{R}F" vertrieben werden, sowie auch bisquaternäre langkettige Ammoniumverbindungen der in der US 4 157 388 A1 beschriebenen Harnstoff-Struktur, die unter dem Namen "Mirapol^{R}A 15" im Handel sind.

Verwiesen wird in diesem Zusammenhang auch auf die in den DE- 25 21 960 A1, 28 11 010 A1, 30 44 738 A1 und 32 17 059 A1 genannten kationaktiven Polymeren sowie in der EP 337 354 A1 auf den Seiten 3 bis 7 beschriebenen Produkte. Es können auch Mischungen verschiedener kationischer Polymerer eingesetzt werden.

Als amphotere Polymere, die alleine oder im Gemisch mit kationischen, nichtionischen oder anionischen Polymeren zum Einsatz gelangen können, seien insbesondere Copolymerisate aus N-Octylacrylamid, (Meth)Acrylsäure und tert.-Butylaminoethylmethacrylat vom Typ "Amphomer^{R}"; Copolymerisate aus Methacryloylethyllbetain und Alkylmethacrylaten vom Typ "Yukaformer^{R}", z. B. das Butylmethacrylat-Copolymere "Yukaformer^{R} Am75"; Copolymerisate aus Carboxylgruppen und Sulfongruppen enthaltenden Monomeren, z. B. (Meth)Acrylsäure und Itaconsäure, mit basische Gruppen, insbesondere Aminogruppen, enthaltenden Monomeren wie Mono- bzw. Dialkylaminoalkyl(meth)acrylaten bzw. Mono-bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US 3,927,199 A1 bekannten Copolymeren genannt.

Besonders geeignete anionische Polymere, sind Vinylalkylether-, insbesondere Methylvinylether/Maleinsäure-Copolymere, die durch Hydrolyse von Vinylether/Maleinsäureanhydrid-Copolymeren entstehen und unter der Handelsbebezeichnung "Gantrez^{R} AN oder ES" vertrieben werden. Diese Polymeren können auch teilverestert sein, beispielsweise "Gantrez^{R} ES 225", der Ethylester eines Ethylvinylether/Maleinsäure-Copolymers, oder der Butyl- oder Isobutylester desselben.

Weitere geeignete anionische Polymere sind insbesondere Vinylacetat/Crotonsäure-Copolymere des Typs "Resyn^{R}"; Natriumacrylat/Vinylalkohol-Copolymere des Typs "Hydagen^{R}F", Natriumpolystyrolsulfonat, z.B. "Flexan^{R} 130"; Ethylacrylat/Acrylsäure/N-tert.-Butylacrylamid-Copolymere des Typs "Ultrahold^{R}"; Vinylpyrrolidon/Vinylacetat/Itaconsäure-Copolymere, Acrylsäure/Acryl-amid-Copolymere bzw. Natriumsalze derselben vom Typ "Reten^{R}"; etc.

Selbstverständlich können in diesen Zusammensetzungen alle in haarkonditionierenden und festigenden Mitteln üblichen und bekannten Stoffe mitverwendet werden, vorausgesetzt, daß sie mit den essentiellen Wirkstoffen nicht interferieren. Solche Stoffe sind beispielsweise oberflächenaktive Mittel, Verdickungsmittel, Perlglanz gebende Mittel, Farbstoffe, Riechstoffe, Konservierungsmittel, etc.

Besonders bevorzugt ist die Mitverwendung von Verdickungsmitteln zur Einstellung der erfindungsgemäß erforderlichen Viskosität.

Geeignete Verdickungsmittel sind insbesondere Polyacrylsäure und deren Salze, bekannt unter der Bezeichnung "Carbomer"; jedoch sind auch weitere Verdickungsmittel wie Hydroxyalkylcellulosen, und andere Kohlenhydrate, z.B. Xanthan Gum, Guar Gum und deren Abwandlungsprodukte, Alginate, Polyvinylpyrrolidon und gegebenenfalls auch anorganische Verdickungsmittel wie Silica oder Magnesiumaluminiumsilikate geeignet.

Der Anteil an Verdickungsmittel ist natürlich abhängig von der gewünschten Viskosität und auch der Art des Verdickungsmittels; der Rahmen liegt dabei zwischen etwa 0,1 und etwa 5 Gew.-%, wobei zu berücksichtigen ist, das auch viele Filmbildner selbst eine verdickende Wirkung aufweisen.

Eine generelle Übersicht über die allgemeine Zusammensetzung von Haarfestigern findet sich in der Monographie von K.Schrader, Grundlagen und Rezepturen der Kosmetika, 2. Aufl. (1989), S. 737-760.

Die Zusammensetzung B stellt eine wäßrige Emulsion, insbesondere eine gelartige Mikroemulsion, dar, und enthält als prinzipielle Bestandteile Öle bzw. Fette, Emulgatoren und Wasser.

Als Öle sind prinzipiell alle für kosmetische Zwecke bekannten natürlichen und synthetischen Öle geeignet, besonders bevorzugt ist Mineralöl /Paraffinöl).

Jedoch können natürlich auch andere Öle, Fette und Wachse, Kompatibilität mit den weiteren Rezepturbestandteilen vorausgesetzt, (mit-)verwendet werden, z.B. Sojaöl, Olivenöl, Avocadoöl, Erdnußöl, Sonnenblumenöl, Sesamöl, Mandelöl, Weizenkeimöl, Jojobaöl, Pfirsichöl, Spermöl, Nachtkerzenöl sowie Wachse wie Paraffinwachs, Bienenwachs, Carnaubawachs, Candilillawachs und auch Fettsäureester wie Myristylmyristat, Cetylstearart, Decyloleat, etc. Wichtig ist, daß diese Substanzen keinen polaren Charakter aufweisen.

Der Anteil der Öle bzw. Fette in den Mikroemulsionen liegt bei etwa 7 bis etwa 30, vorzugsweise etwa 10 bis 25 Gew.-% der Gesamtzusammensetzung.

Als Emulgatoren werden vorzugsweise nichtionische und gegebenenfalls bestimmte anionische Emulgatoren verwendet, deren Menge ungefähr zwischen 10 und 30 Gew.-%, berechnet auf die Gesamtzusammensetzung, liegt.

Beispielhafte nichtionische Emulgatoren sind Fettsäurepolyglykolester, Fettalkoholethoxylate und Glycerinfettsäureesterethoxylate; geeignete anionische Emulgatoren sind insbesondere o-Phosphorsäureester, z.B. Fettalkoholetherphosphate.

Durch den Zusatz von Fettalkoholen wie Stearylalkohol und Cetylalkohol oder nichtionischen Emulgatoren mit niedrigem HLB-Wert zur amphiphilen Phase läßt sich die Viskosität der Emulsion erhöhen.

Ein weiterer bevorzugter Bestandteil der erfindungsgemäß als Zusammensetzung B verwendeten Mikroemulsionen sind Polyole wie Glycerin, Sorbit, Propandiol und Butandiol, vorzugsweise in einer Menge von etwa 5 bis etwa 30 Gew.-%, insbesondere etwa 10 bis etwa 25 Gew.-%, berechnet auf die Gesamtzusammensetzung der Emulsion.

Zur Vermeidung von Wiederholungen wird auch hier auf K. Schrader, 1.c., S. 387 bis 525, verwiesen, wo die Bestandteile von Emulsionen und deren Herstellung ausführlich beschrieben ist, insbesondere aufs das Kapitel "Mikroemulsionen" auf den Seiten 522 bis 525.

Auf diese Offenbarung wird ausdrücklich Bezug genommen.

Die getrennte Aufbewahrung der Zusammensetzungen A und B nach der Erfindung kann vorzugsweise in einem Zweikammerbehältnis bekannter Struktur erfolgen, woraus dann die Zusammensetzungen nacheinander entnommen und im Verhältnis von etwa 1:1 gemischt werden.

Beim Vermischen brechen die Gelstrukturen sowohl der mittelviskosen Konditioniermittel- und Festigerzusammensetzung A als auch der Mikroemulsion B; das Produkt wird niedrigviskos und kann gleichmäßig auf dem Haar verteilt werden.

Eine weitere zweckmäßige Form der Konfektionierung beider Zusammensetzungen besteht in einer Anordnung als Zweierpack von zwei Behältnissen.

Grundsätzlich ist natürlich auch eine Applikation aus zwei nicht miteinander verbundenen Produktbehältnissen zur Vermischung auf der Hand des Verbrauchers bzw. der Verbraucherin möglich.

Durch das folgende Anwendungsbeispiel wird die Erfindung näher erläutert.

| **Zusammensetzung A** | |
|---|---|
| **Styling Lotion** | |
| Polyacrylsäure (Carbopol^{R} ETD 2001) | 0,60 (Gew.-%) |
| Vinylpyrrolidon/Vinylacetat-Copolymerisat | 7,00 |
| Isopropylalkohol | 7,00 |
| Ethanol | 5,50 |
| Organopolysiloxan (Polysilicone-9) | 0,50 |
| PEG-40-hydriertes Rizinusöl | 0,25 |
| UV-Absorber | 0,50 |
| 2-Amino-2-methyl-1-propanol | 0,40 |
| Konservierungsmittel (Parabene) | 0,10 |
| Parfum | 0,25 |
| Wasser | ad 100,00 |
| | |
| Viskosität bei 20°C (Brookfield Spindel T-D, 5UpM): | |
| Etwa 100 000 mPa.s | |

| **Zusammensetzung B** | |
|---|---|
| **Gel-Wachs (Mikroemulsion)** | |
| Oleth-5 | 8,00 (Gew.-%) |
| Mineralöl | 13,00 |
| Sorbit | 13,00 |
| Oleth-5-phosphat | 6,50 |
| 1,3-Butandiol | 5,00 |
| Glycerin | 4,00 |
| Ceteareth-20 | 3,00 |
| Tetrahydroxypropylethylendiamin | 2,90 |
| Octylmethoxycinnamat | 0,80 |
| Cetrimoniumchlorid | 0,40 |
| Konservierungsmittel (Parabene) | 0,60 |
| UV-Absorber | 0,20 |
| Polysilicone-9 | 0,05 |
| Panthenol | 0,05 |
| Parfum | 0,60 |
| Wasser | ad 100,00 |
| | |
| Viskosität bei 20°C (Brookfield Spindel T-E, 5UpM): | |
| Etwa 600 000 mPa.s. | |

Die Mischung der Zusammensetzungen A und B im Verhältnis von etwa 1:1 ergab ein dünnflüssiges Produkt mit einer Viskosität von etwa 430 mPa.s bei 20°C (Brookfield Spindel CP-51, 20 UpM), das sich leicht und gleichmäßig im Haar verteilen läßt und diesem Festigkeit, Volumen und Glanz verleiht.

## Patentansprüche

1. Haarbehandlungsmittel, bestehend aus zwei bis zu ihrer Anwendung getrennt gehaltenen Zusammensetzungen A und B, wobei die Zusammensetzung A in wäßriger oder wäßrig-alkoholischer Grundlage mindestens ein filmbildendes natürliches oder synthetisches Polymeres enthält und eine Viskosität zwischen 25 000 und 150 000 mPa.s bei 20°C, gemessen im Brookfield-Viskosimeter mit einer Spindel T-D bei 5 UpM, aufweist, und die davon getrennt gehaltene Zusammensetzung B als gelförmige, Fette bzw. Öle und Emulgator(en) enthaltende wäßrige Emulsion mit einer Viskosität zwischen 300 000 und 1 000 000 mPa.S bei 20°C, gemessen im Brookfield-Viskosimeter mit einer Spindel T-E bei 5 UpM, vorliegt, und bei der Vermischung beider Zusammensetzungen ein niedrigviskoses Produkt mit einer Viskosität zwischen 100 und 1500 mPa.s bei 20°C, gemessen im Brookfield-Viskosimeter mit einer Spindel CP-51 bei 20 UpM, erhalten wird.

2. Haarbehandlungsmittel nach Anspruch 1, dadurch gekennzeichnet, daß die haarfestigende Zusammensetzung A eine Viskosität zwischen 50 000 und 125 000 mPa.s bei 20°C, und die Zusammensetzung B eine Viskosität zwischen 400 000 und 800 000 mPa.s bei 20°C, gemessen unter den in Anspruch 1 definierten Bedingungen, aufweist.

3. Verfahren zur Haarbehandlung, insbesondere zur Konditionierung und Festigung von menschlichem Haar, dadurch gekennzeichnet, daß auf das Haar ein niedrigviskoses Produkt mit einer Viskosität zwischen 100 und 1500 mPa.s (gemessen bei 20°C im Brookfield-Viskosimeter mit einer Spindel CP-51 bei 20 UpM) aufgebracht und auf diesem gleichmäßig verteilt wird, das durch Vermischen einer mittelviskosen, mindestens ein filmbildendes Polymer enthaltenden wäßrigen oder wäßrig-alkoholischen Haarfestiger-Zusammensetzung mit einer Viskosität zwischen 25 000 und 150 000 mPa.s (gemessen bei 20°C im Brookfield-Viskosimeter mit einer Spindel T-D bei 5 UpM) und einer hochviskosen gelförmigen, Fette bzw. Öle und Emulgator(en) enthaltenden wäßrigen Emulsion mit einer Viskosität zwischen 300 000 und 1 000 000 mPa.s (gemessen bei 20°C im Brookfield-Viskosimeter mit einer Spindel T-E bei 5UpM) erhalten wurde.

## Claims

1. Composition for the treatment of human hair, consisting of two Compositions A and B, kept separate from each other until use, whereby the Composition A in an aqueous or hydro-alcoholic carrier material comprises at least one film-forming natural or synthetic polymer having a viscosity from 25,000 to 150,000 mPa.s at 20°C, measured in a Brookfield Viscosimeter with a T-D spindle at 5 rpm, and Composition B, held separate from Composition A, is a gel-like aqueous emulsion, containing fats or oils and one or more emulsifier(s), having a viscosity from 300,000 to 1,000,000 mPa.s at 20°C, measured in a Brookfield Viscosimeter with a T-E spindle at 5 rpm, and after mixture of the two Compositions a low-viscosity product is obtained, having a viscosity from 100 to 1,5000 mPa.s at 20°C, measured in a Brookfield Viscosimeter with a CP-51 spindle at 20 rpm.

2. Composition for the treatment of human hair according to claim 1, characterized in that Composition A has a viscosity from 50,000 to 125,000 mPa.s at 20°C, and Composition B has a viscosity from 400,000 to 800,000 mPa.s at 20°C, measured under the conditions as defined in claim 1.

3. Process for the treatment of human hair, in particular for its conditioning and setting, characterized in that a low viscosity product with a viscosity from 100 to 1,500 mPa.s (measured at 20°C in a Brookfield Viscosimeter with a CP-51 spindle at 20 rpm) is applied to human hair and even distributed therein, said product having been obtained by admixture of an aqueous or hydro-alcoholic hair setting composition of a viscosity from 25,000 to 150,000 mPa.s (measured at 20°C in a Brookfield Viscosimeter with a T-D spindle at 5 rpm), comprising at least one film-forming polymer, with a gel-like aqueous emulsion, containing fats or oils and one or more emulsifier(s), of a viscosity from 300,000 to 1,000,000 mPa.s (measured at 20°C in a Brookfield Viscosimeter with a T-E spindle at 5 rpm).

## Revendications

1. Agent de soin capillaire, constitué de deux compositions A et B maintenues séparées jusqu'à leur utilisation, la composition A comportant dans une base aqueuse ou hydroalcoolique au moins un polymère naturel ou synthétique filmogène, d'une viscosité comprise entre 25.000 et 150.000 mPa.s à 20°C, mesurée au viscosimètre Brookfield avec une broche T-D à 5 tours/minute, et la composition B maintenue séparée de la première se présente sous la forme d'une émulsion aqueuse en forme de gel qui comporte des graisses ou des huiles et un ou plusieurs émulsifiants, d'une viscosité comprise entre 300.000 et 1.000.000 mPa.s à 20°C, mesurée au viscosimètre Brookfield avec une broche T-E à 5 tours/minute, et lors du mélange des deux compositions, on obtient un produit de faible viscosité, d'une viscosité comprise entre 100 et 1.500 mPa.s à 20°C, mesurée au viscosimètre Brookfield avec une broche CP-51 à 20 tours/minute.

2. Agent de soin capillaire selon la revendication 1, caractérisé en ce que la composition A de fixation des cheveux présente une viscosité comprise entre 50.000 et 125.000 mPa.s à 20°c, mesurées dans les condition définies dans la revendication 1.

3. Procédé de soin capillaire, en particulier pour le conditionnement et la fixation des cheveux humains, caractérisé en ce qu'un produit de faible viscosité, d'une viscosité comprise entre 100 et 1.500 mPa.s (mesurée à 20°c au viscosimètre Brookfield avec une broche CP-51 à 20 tours/minute) est appliqué sur les cheveux et y est réparti régulièrement, lequel produit a été obtenu par mélange d'une composition de fixation des cheveux, de viscosité moyenne, qui comporte dans une base aqueuse ou hydroalcoolique au moins un polymère naturel ou synthétique filmogène, d'une viscosité comprise entre 25.000 et 150.000 mPa.s (mesurée à 20°C au viscosimètre Brookfield avec une broche T-D à 5 tours/minute) et d'une émulsion aqueuse en forme de gel, de viscosité élevée, comportant des graisses ou des huiles et un ou plusieurs émulsifiants, d'une viscosité comprise entre 300.200 et 1.000.000 mPa.s (mesurée à 20°C au viscosimètre Brookfield avec une broche T-E à 5 tours/minute).
